**Europäisches Patentamt**

**European Patent Office**

**Office européen des brevets**

(19)

(11) Numéro de publication: **0 143 703**
**B1**

(12)

# FASCICULE DE BREVET EUROPÉEN

(45) Date de publication du fascicule du brevet:
**08.04.87**

(21) Numéro de dépôt: **84402355.6**

(22) Date de dépôt: **20.11.84**

(51) Int. Cl.⁴: **C 07 C 53/16,** C 07 C 53/18, C 07 C 51/41 // C07C2/08, C07C11/02

(54) **Nouvelle composition de nickel soluble dans les hydrocarbures et son utilisation.**

(30) Priorité: **29.11.83 FR 8319183**

(43) Date de publication de la demande:
**05.06.85 Bulletin 85/23**

(45) Mention de la délivrance du brevet:
**08.04.87 Bulletin 87/15**

(84) Etats contractants désignés:
**BE DE GB IT NL SE**

(56) Documents cité:
**Néant.**

(73) Titulaire: **INSTITUT FRANCAIS DU PETROLE, 4, Avenue de Bois- Préau, F-92502 Rueil- Malmaison (FR)**

(72) Inventeur: **Provin, Gérard, 11, rue des Marais, F-95460 Ezanville (FR)**
Inventeur: **Forestiere, Alain, Le Prévert - Bâtiment B Route de Lyon, F-69390 Vernaison (FR)**
Inventeur: **Commereuc, Dominique, 32, rue Abel Vacher, F-92190 Meudon (FR)**

## Description

L'objet de la présente invention est un procédé de préparation d'une nouvelle composition du nickel soluble dans les hydrocarbures et contenant un anion halogénoacétate.

On sait que les carboxylates de nickel bivalent dérivés d'acides à chaîne courte, c'est-à-dire comportant moins de 5 atomes de carbone tels que les acétate, propionate, butyrate et leurs dérivés halogénés tels que chloroacétate, dichloroacétate, trichloroacétate, trifluoroacétate sont, dans des conditions normales de température, pratiquement insolubles dans les hydrocarbures. Par contre les carboxylates de nickel bivalent dérivés d'acides à longues chaînes, c'est-à-dire comportant plus de 5 atomes de carbone et de préférence comportant une ramification, sont solubles dans les hydrocarbures et même totalement miscibles avec eux; tel est le cas par exemple de l'éthyl-2 hexanoate de nickel. Il a été décrit dans le brevet français n° 2.464.243 un moyen de solubiliser l'anion halogénoacétate lié au nickel grâce à la préparation d'un sel mixte dans lequel le nickel est lié par une de ses valences à l'anion halogénoacétate et par l'autre à l'anion d'un acide carboxylique à longue chaîne, c'est-à-dire comportant au moins 5 atomes de carbone.

Cependant, la présence dans un dérivé halogénoacétique du nickel d'un anion dérivé d'un acide carboxylique à longue chaîne est cause d'un coût additionnel de fabrication et d'autre part présente parfois des inconvénients lors de leur utilisation. C'est ainsi que dans une de ces utilisations, les sels de nickel contenant l'anion halogénoacétate sont mis en réaction avec des halogénures d'alkylaluminium en solution hydrocarbonée pour catalyser la dimérisation des oléfines en phase liquide; un des moyens d'éliminer les composés inorganiques présents dans le milieu en fin de dimérisation consiste en une extraction par une solution aqueuse de soude; la présence lors de cette étape d'un anion carboxylate à longue chaîne conduit, en raison des propriétés tensioactives de son dérivé sodique, à des mousses qui nuisent à la séparation des phases, et à une mauvaise dispersion de l'oxyde de nickel produit par réaction du catalyseur avec la soude.

L'objet de la présente invention est donc de proposer une méthode de préparation d'une nouvelle composition du nickel, soluble dans les hydrocarbures, contenant l'anion halogénoacétate, ladite méthode permettant d'éviter l'emploi d'acide ou d'anion carboxylate à plus de 5 atomes de carbone.

Il a été découvert que l'interaction d'au moins un composé inorganique de nickel bivalent avec un acide halogénoacétique au sein d'un ester d'acide carboxylique conduit à une composition liquide soluble dans les hydrocarbures. Ceci est d'autant plus inattendu que les dérivés inorganiques du nickel qui sont mis en oeuvre et que les halogénoacétates de nickel à la formation desquels on peut s'attendre, sont totalement insolubles dans les hydrocarbures dans les conditions normales de température.

Le composé minéral du nickel utilisé selon l'invention, est par exemple un carbonate, un bicarbonate, un carbonate basique (hydroxycarbonate), un hydroxyde ou un oxyde.

L'acide halogénoacétique est par exemple l'acide monochloracétique, monofluoroacétique, dichloroacétique, trichloroacétique, difluoroacétique ou trifluoroacétique.

Les esters d'acides carboxyliques utilisés selon l'invention répondent à la formule générale $R_1COOR_2$ dans laquelle $R_1$ et $R_2$ sont des radicaux hydrocarbyl, $R_1$ pouvant aussi être l'hydrogène. Plus particuliérement $R_1$ et $R_2$ peuvent être chacun un groupement alkyl ramifié ou linéaire comportant 1 à 20 atomes de carbone, de préférence 1 à 5 atomes de carbone, ou un groupement aryl comportant 6 à 9 atomes de carbone, $R_1$ pouvant être également l'hydrogène, par exemple l'acétate de méthyle, le formiate de méthyle, l'acétate de n butyle, l'acétate d'isobutyle, le propionate de méthyle, le formiate d'isopropyle.

Selon l'invention, l'acide halogénoacétique est mis en oeuvre dans la proportion de 0,1 à 2 moles, de préférence de 0,5 à 1,5 moles par atome de nickel engagé.

L'ester tel que défini ci-dessus est mis en oeuvre en quantité telle que pour 1 atome gramme de nickel engagé, on utilise entre 0,1 et 10 litres d'ester et de préférence entre 0,2 et 2 litres d'ester.

Au mélange des 3 composants ci-dessus définis, on peut également ajouter un acide carboxylique non halogéné renfermant au moins 3 atomes de carbone tel que l'acide butyrique, l'acide laurique, l'acide éthyl-2 hexanoïque, l'acide stéarique. Mais contrairement à ce qui est revendiqué dans le brevet français n° 2.464.243, l'addition de cet acide n'est pas indispensable pour obtenir la solubilité dans les hydrocarbures et d'autre part, il est utilisé dans un rapport molaire acide/nickel inférieur à 1, de préférence 0,1 à 0,9 mole d'acide carboxylique par atome de nickel.

Selon un mode préféré de réalisation de l'invention, on introduit, tout en agitant vigoureusement, l'acide halogénoacétique dans le mélange préalablement formé par le composé du nickel et l'ester.

La température de mélange est comprise entre l'ambiante et le point d'ébullition de l'ester sous la pression choisie, de préférence à reflux et en éliminant le gaz carbonique et l'eau éventuellement formés au cours de la réaction. La réaction est poursuivie jusqu'à solubilisation complète du nickel, disparition totale de l'acide halogénoacétique libre et fin de formation d'eau et/ou de gaz carbonique. L'introduction éventuelle de l'acide carboxylique non halogéné peut se faire soit avant l'introduction de l'acide halogénoacétique soit à tout moment de la réaction et même à la fin.

On peut réaliser la réaction en présence d'un hydrocarbure tel que le pentane, l'hexane, l'heptane, l'octane ou leurs isomères et leur mélange ou le benzène, le toluène, les xylènes; l'hydrocarbure peut alors être introduit en quantité variable, en début, en cours ou en fin de réaction. L'hydrocarbure peut éventuellement servir à éliminer l'eau de réaction.

L'invention concerne aussi les compositions catalytiques renfermant (a) une composition de nickel soluble telle que décrite ci-dessus et (b) un halogénure d'hydrocarbylaluminium, ce dernier en proportion préférée de 1 à 50 moles par atome de nickel, de préférence 2 à 20 moles par atome de nickel.

L'invention concerne aussi l'emploi de la composition catalytique précitée comme catalyseur de dimérisation d'oléfine en phase liquide, à une température préférée de 0 à 60° C.

L'oléfine est, par exemple, l'éthylène, le propylène ou un butène.

La composition catalytique est utilisée de préférence en proportion correspondant à 5 à 500 parties par million en poids.

Les exemples suivants illustrent l'invention sans en limiter la portée:

## Exemple 1

On porte à reflux pendant 2 heures en éliminant l'eau qui se forme, un mélange de 10g ($8.10^{-2}$ mole) de carbonate basique de nickel $NiCO_3$, $Ni(OH)_2$, $H_2O$ avec 150 $cm^3$ d'acétate de butyle et 6 $cm^3$ ($8.10^{-2}$ mole) d'acide trifluoroacétique.

On obtient une solution micellaire verte qui peut être diluée à volonté par l'heptane sans qu'on observe de précipitation.

## Exemple 2

On répète l'exemple 1 en ajoutant en plus 4,4g ($5.10^{-2}$ mole) d'acide butyrique.

La solution obtenue de couleur verte, peut être diluée à volonté par l'heptane sans qu'on observe de précipitation.

## Exemple 3

On répète l'exemple 2 à ceci près qu'on a remplacé l'acide trifluoroacétique par $8.10^{-2}$ mole d'acide trichloracétique. On obtient le même type de solution micellaire qui contient la presque totalité du nickel introduit et qui peut être diluée à volonté dans les hydrocarbures.

## Exemple 4

Cet exemple ne fait pas partie de l'invention, mais illustre la nécessité d'opérer dans un ester pour obtenir le nickel en solution.

On a porté à reflux dans 200 $cm^3$ d'hexane un mélange de 10g du même carbonate de nickel et de 6 $cm^3$ d'acide trifluoroacétique. On observe un dégagement rapide de gaz carbonique tandis que le milieu reste totalement incolore et que se forme un composé insoluble qui a été caractérisé comme étant du bistrifluoroacétate de nickel.

## Exemple 5

On a opéré comme dans l'exemple 2 à ceci près qu'on a remplacé l'acétate de butyle par 150 $cm^3$ de formiate de méthyle. On a obtenu une solution verte contenant la plus grande partie du nickel introduit.

## Exemple 6

On a opéré comme dans l'exemple 2 à ceci près qu'on a remplaçé l'acétate de butyle par du butyrate d'éthyle. On a obtenu une solution verte contenant la totalité du nickel introduit et qui peut être diluée à volonté dans de l'heptane.

## Exemple 7

On a utilisé la solution obtenue dans l'exemple 1 pour dimériser les butènes.

Dans un système constitué de 2 réacteurs de 0,25 litre, montés en série et dont on peut assurer la régulation thermique, on introduit en continu à 45°C: 100g/h d'une coupe d'hydrocarbures en $C_4$ contenant 72% de n-butènes; 0,1m M/h de nickel sous la forme de la composition de l'exemple 1 dissoute dans de l'heptane et 1,5 m M/h de dichloroéthylaluminium dissous dans de l'heptane. L'effluent hydrocarboné du 2ème étage, dans le but d'éliminer les composants minéraux du catalyseur, est traité oar une solution aqueuse de soule à 18% en poids et est ensuite lavé en continu par de l'eau, dans 2 réacteurs en série également d'un volume de 0,25 litre chacun.

On n'observe pas de difficulté de décantation des phases en présence, et on recueille la phase hydrocarbonée correctement déminéralisée. L'oxyde de nickel qui précipite sous l'action de la solution de soude, est entièrement dispersé dans celle-ci et peut donc être aisément éliminé de la phase hydrocarbonée.

Dans un essai comparatif pour lequel on avait utilisé le sel mixte trifluoracétate-éthyl-2 hexanoate de nickel à la place de la composition de l'exemple 2 on a observé la formation de mousses abondantes dans l'étape de lavage à l'eau et qui a empéché toute décantation correcte. En outre l'oxyde de nickel qui avait précipité se retrouve à l'interface entre la solution aqueuse de soude et la phase hydrocarbonée, ce qui rend difficile l'élimination du nickel.

**Exemple 8**

Dans un système réactionnel constitué de 2 étages en série d'un volume de 9 litres maintenus à 40° C et sous une pression de 2 MPa, on introduit en continu 1000 g/h d'une coupe contenant 95 % de propylène, 5,5 m M/h de dichloroéthylaluninium en solution dans de l'heptane et 0,55 m M/h de nickel sous la forme de la composition de l'exemple 2 dissoute dans de l'heptane. En régime stationnaire, la conversion observée dans le 1er étage est de 90,1 % et celle de l'ensemble des étages de 96,5 %. L'effluent est traité par une solution aqueuse de soude à 20 % puis par de l'eau. On observe une bonne décantation entre les phases hydrocarbonée et aqueuse. L'oxyde de nickel qui précipite sous l'action de la solution de soude, est entiérement dispersé dans celle-ci et peut donc être aisément éliminé de la phase hydrocarbonée.

**Exemple 9**

On a répété l'exemple 8 en remplaçant la composition catalytique de l'exemple 2 par celle de l'exemple 6. On a obtenu une conversion de 89 % dans le 1er étage et de 95,5 % pour l'ensemble des étages. La séparation est aussi bonne que dans l'exemple 8.

**Revendications**

1. Nouvelle composition de nickel soluble dans les hydrocarbures et caractérisée en ce qu'elle est préparée par l'intéraction:
- d'au moins un composé minéral de nickel bivalent;
- d'au moins un acide halogénoacétique;
et d'au moins un ester de formule générale $R_1COOR_2$ dans laquelle $R_1$ est l'hydrogène ou un radical hydrocarbyl et $R_2$ est un radical hydrocarbyl.

2. Composition selon la revendication 1 dans la préparation de laquelle l'acide halogénoacétique est mis en réaction à raison de 0,1 à 2 moles par atome de nickel du composé minéral et l'ester est en proportion de 0,1 à 10 litres par atome de nickel du composé minéral.

3. Composition selon la revendication 1 dans la préparation de laquelle l'acide halogénoacétique est mis en réaction à raison de 0,5 à 1,5 mole et l'ester en proportion de 0,2 à 2 litres par atome de nickel du composé minéral.

4. Composition selon l'une quelconque des revendications 1 à 3, dans la préparation de laquelle le composé minéral de nickel est un carbonate, un bicarbonate, un carbonate basique, un oxyde ou un hydroxyde.

5. Composition selon l'une quelconque des revendications 1 à 3 dans la préparation de laquelle le composé minéral de nickel est un carbonate basique de nickel.

6. Composition selon l'une quelconque des revendications 1 à 5 dans la préparation de laquelle l'acide halogenoacétique est l'acide trifluoroacétique.

7. Composition selon l'une quelconque des revendications 1 à 6 dans la préparation de laquelle un acide carboxylique non halogéné renfermant au moins 3 atomes de carbone est également mis en réaction, la proportion de cet acide étant de 0,1 à 0,9 mole par atome de nickel.

8. Composition selon l'une des revendications 1 à 7, dans laquelle $R_1$ est l'hydrogène ou un radical alkyle de 1 à 5 atomes de carbone et $R_2$ est un radical alkyle de 1 à 5 atomes de carbone.

9. Composition catalytique, caractérisée en ce qu'elle comprend
(a) une composition selon l'une des revendications 1 à 8 et
(b) un halogènure d'hydrocarbylaluminium.

10. Utilisation de la composition selon la revendication 9, comme catalyseur de dimérisation d'oléfine en phase liquide.

**Patentansprüche**

1. Neue in Kohlenwasserstoffen lösliche Nickel-Zusammensetzung, dadurch gekennzeichnet, daß sie hergestellt ist durch Umsetzung von:
- zumindest einer anorganischen Verbindung des zweiwertigen Nickels,
- zumindest einer Halogenessigsäure,
- und zumindest einem Ester der allgemeinen Formel $R_1COOR_2$, worin $R_1$ Wasserstoff oder einen Kohlenwasserstoffrest und $R_2$ einen Kohlenwasserstoffrest bedeuten.

2. Zusammensetzung nach Anspruch 1, dadurch gekennzeichnet, daß bei ihrer Herstellung die Halogenessigsäure im Verhältnis 0,1 bis 2 Mol pro Atom Nickel der anorganischen Verbindung umgesetzt wird und der Ester in einer Menge von 0,1 bis 10 Liter pro Atom Nickel der anorganischen Verbindung vorliegt.

3. Zusammensetzung nach Anspruch 1, dadurch gekennzeichnet, daß bei ihrer Herstellung die Halogenessigsäure in einem Verhältnis von 0,5 bis 1,5 Mol umgesetzt wird und der Ester in einer Menge von 0,2 bis 2 Liter pro Atom Nickel der anorganischen Verbindung vorliegt.

4. Zusammensetzung nach einem der Ansprüche 1 bis 3, dadurch gekennzeichnet, daß bei ihrer Herstellung die anorganische Nickelverbindung ein Carbonat, Bicarbonat, basisches Carbonat, Oxid oder ein Hydroxyd ist.

5. Zusammensetzung nach einem der Ansprüche 1 bis 3, dadurch gekennzeichnet, daß bei ihrer Herstellung die anorganische Nickelverbindung ein basisches Nickelcarbonat ist.

6. Zusammensetzung nach einem der Ansprüche 1 bis 5, dadurch gekennzeichnet, daß bei der Herstellung die Halogenessigsäure Trifluoressigsäure ist.

7. Zusammensetzung nach einem der Ansprüche 1 bis 6, dadurch gekennzeichnet, daß bei ihrer Herstellung eine nicht halogenierte Carbonsäure mit zumindest 3 Kohlenstoffatomen ebenfalls umgesetzt wird, wobei der Mengenanteil dieser Säure 0,1 bis 0,9 Mol pro Atom Nickel beträgt.

8. Zusammensetzung nach einem der Ansprüche 1 bis 7, dadurch gekennzeichnet, daß in ihr $R_1$ Wasserstoff oder einen Alkylrest mit 1 bis 5 Kohlenstoffatomen und $R_2$ einen Alkylrest mit 1 bis 5 Kohlenstoffatomen bedeuten.

9. Katalytische Zusammensetzung, dadurch gekennzeichnet, daß sie enthält
(a) eine Zusammensetzung nach einem der Ansprüche 1 bis 8,
und
(b) ein Halogenid eines Aluminiumkohlenwasserstoffes.

10. Verwendung der Zusammensetzung nach Anspruch 9 als Dimerisierungskatalysator für Olefin in flüssiger Phase.

## Claims

1. A new nickel composition, soluble in hydrocarbons, characterized as being obtained by reacting:
- at least one divalent nickel inorganic compound;
- at least one halogenoacetic acid;
- and at least one ester of the general formula $R_1COOR_2$, wherein $R_1$ is hydrogen or a hydrocarbyl radical and $R_2$ a hydrocarbyl radical.

2. A composition according to claim 1, whose manufacture comprises the use of halogenoacetic acid in a proportion of 0.1 - 2 moles per nickel atom of the inorganic compound and ester in a proportion of 0.1-10 liters per nickel atom of the inorganic compound.

3. A composition according to claim 1, whose manufacture comprises the use of halogenoacetic acid in a proportion of 0.5 - 1.5 mole and ester in a proportion of 0.2 - 2 liters per nickel atom or the inorganic compound.

4. A composition according to any one of claims 1 to 3, whose manufacture comprises, as nickel inorganic compound, a carbonate, bicarbonate, basic carbonate, oxide or hydroxide.

5. A composition according to any one of claims 1 to 3, whose manufacture comprises, as nickel inorganic compound, a nickel basic carbonate.

6. A composition according to any one of claims 1 to 5, whose manufacture comprises, as halogenoacetic acid, trifluoroacetic acid.

7. A composition according to any one of claims 1 to 6, whose manufacture further comprises the reaction of a non-halogenated carboxylic acid having at least 3 carbon atoms, in a proportion of 0.1 - 0.9 mole per nickel atom.

8. A composition according to one of claims 1 to 7, wherein $R_1$ is hydrogen or an alkyl radical having 1 - 5 carbon atoms, and $R_2$ is an alkyl radical having 1 - 5 carbon atoms.

9. A catalytic composition characterized in that it comprises
a) a composition according to one of claims 1 to 8, and
b) a hydrocarbylaluminum halide.

10. The use of the composition of claim 9 as catalyst for dimerizing olefins in liquid phase.